# EUROPÄISCHE PATENTSCHRIFT

(11) **EP 1 852 092 B1**
(45) Veröffentlichungstag und Bekanntmachung des Hinweises auf die Patenterteilung: **15.02.2017**
(21) Anmeldenummer: 07008306.8
(22) Anmeldetag: 24.04.2007
(51) Int. Cl.: A61F 2/76, A61F 2/30, A61F 2/50, A61F 2/68, A61F 2/58, A61F 2/78

(54) **Verriegelbares Prothesengelenk**
Latchable prosthetic joint
Articulation prothétique verrouillable

(30) Priorität: 03.05.2006 DE 102006020777
(43) Veröffentlichungstag der Anmeldung: 07.11.2007
(73) Patentinhaber: Otto Bock HealthCare IP GmbH & Co. KG, 37115 Duderstadt (DE)
(72) Erfinder: Bertels, Thomas, 37115 Duderstadt (DE); Groß, Tobias, 37308 Günterode (DE)
(74) Vertreter: Stornebel, Kai

(56) Entgegenhaltungen:
- WO-A-97/10781
- GB-A- 1 386 942
- US-A- 2 812 961
- US-A- 5 913 901
- US-A1- 2004 015 240

## Beschreibung

Die Erfindung betrifft ein Prothesengelenk, insbesondere für eine Handprothese, mit einer Basis und einem der Basis zugeordneten Kopplungselement zur Befestigung an einem Körperglied oder einem Prothesenteil sowie mit einer mit der Basis um zumindest eine Achse verschwenkbar verbundene Aufnahmeeinrichtung für eine Protheseneinrichtung, insbesondere eine Kunsthand, wobei eine Verriegelungseinrichtung die Aufnahmeeinrichtung gegenüber der Basis blockiert.

Aus dem Stand der Technik sind Prothesengelenke bekannt, die eine Verschwenkung zweier Prothesenteile gegeneinander ermöglichen und die zueinander sperrbar sind. Dabei handelt es sich in der Regel um einfache, einachsige Gelenke, die in ihrer jeweiligen Winkelstellung, gegebenenfalls in diskreten Winkelschritten, zueinander fixierbar sind, indem eine Verriegelungseinrichtung die beiden Prothesenteile blockiert.

Verriegelungs- oder Blockiereinrichtungen können jedoch auch bei motorisch angetriebenen Protheseneinrichtungen vorgesehen sein, insbesondere, um eine Energieersparnis zu erzielen, wenn ein Prothesenteil in einer fest eingestellten Position unter Belastung gehalten werden muss.

Wird die Verriegelung aufgelöst und befindet sich das distale Prothesenteil in einem entriegelten Zustand, fällt es gravitationsgetrieben nach unten. Dieses Herunterfallen ist in der Regel nicht kontrollierbar, insbesondere bei nicht angetriebenen Protheseneinrichtungen. So tritt bei Prothesenhänden das Problem auf, dass die Kunsthand nach der Entriegelung der Verriegelungseinrichtung ungedämpft nach unten fällt. Gleiches kann bei Unterarmprothesen mit einem Prothesengelenk im Ellbogenbereich geschehen; analog sieht die Situation bei Fuß- oder Unterschenkelprothesen aus.

Die US 5,913,901 beschreibt ein Prothesenfußgelenk, das in seiner Winkeleinstellung zur Anpassung einer unterschiedlichen Absatzhöhe einstellbar ist. An einem Basisteil sind Zahnsegmentplatten schwenkbar gelagert. Diese Zahnsegmentplatten greifen in einem Verriegelungszustand in eine korrespondierende Verzahnung einer Aufnahmeeinrichtung ein, an der der übrige Prothesenfuß befestigt werden kann. Die schwenkbaren Zahnsegmentplatten können über einen Betätigungsmechanismus außer Eingriff mit der Verzahnung gebracht werden, so dass sich eine schrittweise Winkelverstellbarkeit verwirklichen lässt. Die Betätigungseinrichtung ist federbelastet, so dass die Zahnsegmentplatten elastisch in Richtung auf die Verzahnung vorgespannt sind. Sobald die Zahnsegmentplatten außer Eingriff mit der Verzahnung gebracht worden sind, kann der Prothesenfuß bzw. die Aufnahmeeinrichtung frei gegenüber dem Basisteil verschwenkt werden.

Die US 2004/0015240 A1 beschreibt eine Handprotheseneinrichtung mit einer Basisplatte und einer schwenkbar daran gelagerten halbkreisförmigen Drehaufnahme. Die Drehaufnahme weist ein Aufnahmeeinrichtung für eine Prothesenhand auf. Ein Trommelelement weist eine Vielzahl an Schlitzen auf, in die eine Verriegelungsplatte, die gleitend an der Basisplatte gelagert ist, eingreifen kann. Die Verriegelungsplatte ist über eine Feder in Verriegelungsrichtung vorgespannt, so dass in der verriegelten Stellung die gleitende Verriegelungsplatte in Eingriff mit dem halbkreisförmigen Element steht und eine formschlüssige Verriegelung bewirkt. Wenn der Prothesenträger die Verriegelungsplatte eindrückt, wird die Platte außer Eingriff mit den Schlitzten gebracht und eine freie Drehung kann bewirkt werden.

Die US 4,846,842 A1 betrifft ein verriegelbares Gelenk einer Prothese, das zwei Gelenkelemente aufweist, die zueinander verdreht werden können. Die Gelenkelemente bestehen aus zwei Trägerarmen, zwischen den eine Platte mit Ausnehmungen angeordnet ist, in die eine Verriegelungsscheibe eingreifen kann. Zwischen beiden Gelenkelementen ist eine Schlingfeder angeordnet, die eine Vorspannung in Extensionsrichtung erzeugt. Wird die Verriegelungsscheibe geöffnet und außer Eingriff mit korrespondierenden Aufnahmen gebracht, werden die Gelenkelemente in eine Extensionsstellung gebracht.

Die US 2,812,961 beschreibt eine Verriegelungseinrichtung für ein Prothesengelenk mit einem Verriegelungsschlitten, der an seinem vorderen Ende einen Verriegelungszahn aufweist. Der Verriegelungsschlitten ist in einem Gehäuseteil angeordnet, das an einer Haltelasche befestigt ist. In dem Gehäuse ist ein zweites Gelenkteil angeordnet, das Ausnehmungen auf seinem äußeren Umfang aufweist, in die der Zahn des Verriegelungsschlittens einführbar ist. Gegen einen Fehler kann der Verriegelungsschlitten zurückgezogen werden, um eine freie Beweglichkeit des Gelenkes zu erreichen. Der Verriegelungsschlitten kann in der inaktiven Position über eine Halteklinke in dem Gehäuse gehalten werden.

Die WO98/30177 A1 beschreibt eine Ellenbogenprothese mit einem Unterarmelement zum Aufnehmen einer Handprothese. Der Ellenbogen kann in einer bestimmten Stellung über einen Verriegelungsstift verriegelt werden. Nach dem Entriegeln kann der Unterarm über einen Kabelzugmechanismus bei Unterstützung durch eine Anhebeunterstützungsfeder angehoben werden.

Aufgabe der vorliegenden Erfindung ist es, ein Prothesengelenk bereitzustellen, das dem Träger ein erhöhtes Maß an Komfort und ein möglichst natürliches Erscheinungsbild der Prothese bietet.

Erfindungsgemäß wird diese Aufgabe durch ein Prothesengelenk mit den Merkmalen des Anspruchs 1 gelöst. Vorteilhafte Ausgestaltungen und Weiterbildungen der Erfindung sind in den Unteransprüchen beschrieben.

Das Prothesengelenk, insbesondere für eine Handprothese, mit einer Basis und einem der Basis zugeordneten Kopplungselement zur Befestigung an einem Körperglied oder einem Prothesenteil sowie mit einer mit der Basis um zumindest eine Achse verschwenkbar verbundene Aufnahmeeinrichtung einer Protheseneinrichtung, insbesondere eine Kunsthand, wobei eine Verriegelungseinrichtung die Aufnahmeeinrichtung gegenüber der Basis blockiert, sieht vor, dass ein Rückhalteelement elastisch gegen die Aufnahmeeinrichtung vorgespannt ist und diese mit einer Haltekraft beaufschlagt. Durch diese Haltekraft wird verhindert, dass die Aufnahmeeinrichtung, die das distale Prothesenteil, bei einer Handprothese die Kunsthand, trägt, unmittelbar nach dem Entriegeln der Verriegelungseinrichtung herunterklappt. Insbesondere bei Handprothesen kann die entriegelnde Hand nicht zur Abstützung der Kunsthand eingesetzt werden, da diese gerade mit der Entriegelung der Verriegelungseinrichtung beschäftigt ist. Auch bei anderen Anwendungen kann es hilfreich sein, wenn das Prothesenteil nicht unmittelbar nach der Entriegelung kraftlos geschaltet ist, sondern wenn über eine Haltekraft dieses entweder in der Position gehalten wird, in der die Entriegelung stattfand oder wenn nur eine langsame Absenkung gegen die Schwerkraft stattfindet. Die Erfindung sieht weiterhin vor, dass die Aufnahmeeinrichtung Rastelemente aufweist, in die das Rückhalteelement eingreift, wobei die Rastelemente dergestalt ausgebildet sind, dass das Rückhalteelement bzw. die Rückhalteelemente in diese hinein und wieder hinaus gleiten können. Die Rastelemente dienen einerseits zur Einstellung diskreter Winkelwerte und andererseits zur Verringerung der notwendigen Vorspannkraft, um die Protheseneinrichtung zusammen mit der Aufnahmeeinrichtung in Position zu halten. Dazu sind die Rastelemente bevorzugt als Ausnehmungen, Einkerbungen oder Vorsprünge ausgebildet, die entweder axial oder radial orientiert sind. Die Orientierung hängt von der Ausrichtung des Rückhalteelementes ab.

Die Vorspannung des Rückhalteelementes kann bevorzugt so eingestellt sein, dass die Aufnahmeeinrichtung, insbesondere zusammen mit der daran befestigten Protheseneinrichtung, entgegen der Schwerkraft in der entriegelten Stellung gehalten wird. Dazu ist die Haltekraft zumindest gleich der Schwerkraft einzustellen, so dass sich zumindest ein Kräftegleichgewicht einstellt, das eine Bewegung um die Schwenkachse verhindert. Die Haltekraft ist dabei bevorzugt so eingestellt, dass die Protheseneinrichtung gerade noch gehalten wird, so dass eine anschließende, gewollte Verstellung bzw. Verschwenkung ohne großen Kraftaufwand möglich ist. Um bei wechselnden Anbauteilen bzw. Protheseneinrichtungen die Haltekraft anpassen zu können, ist die Vorspannung des Rückhalteelementes einstellbar, beispielsweise indem eine Federvorspannung variiert wird.

Das Rückhalteelement ist in einer bevorzugten Ausgestaltung als elastisches Band, insbesondere als Rundschnur ausgebildet, was einerseits eine einfache Fertigung und andererseits eine hinreichend große Haltekraft bei gleichzeitiger Ein- und Ausführbarkeit aus den Ausnehmungen bzw. Einkerbungen ermöglicht.

Alternativ zu einer Ausgestaltung als ein elastisches Band kann das Rückhalteelement auch als ein formstabiles Kontaktteil ausgebildet sein, das an der Aufnahmeeinrichtung anliegt und in Richtung auf die Aufnahmeeinrichtung elastisch gelagert ist. Dies ist insbesondere dann sinnvoll, wenn eine hohe Lebensdauer gefordert und damit einhergehend nur ein geringer Abrieb zulässig ist. Während bei einer Relativbewegung eines elastischen Bandes zu der Aufnahmeeinrichtung das elastische Band relativ schnell verschleißt, wird dies durch Zwischenschaltung eines Kontaktteiles, das elastisch gelagert ist, verhindert. Das Kontaktteil kann als Lagerstift, insbesondere als Stahlstift ausgebildet sein und an der Aufnahmeeinrichtung anliegen bzw. in die Ausnehmungen, Einkerbungen oder Vorsprünge eingreifen bzw. an ihnen anliegen. Grundsätzlich ist auch möglich, eine federbelastete Stahlkugel einzusetzen.

Die Verriegelungseinrichtung kann als ein sogenanntes "Push-Push-Element" ausgebildet sein, dass die Aufnahmeeinrichtung formschlüssig gegenüber der Basis verriegelt. Wird die Verriegelungseinrichtung einmal betätigt, findet eine formschlüssige Verriegelung in einer bestimmten, diskreten Position statt. Bei einer erneuten Betätigung der Verriegelungsvorrichtung wird die formschlüssige Verriegelung aufgehoben und eine Beweglichkeit entgegen der Haltekraft durch das Rückhalteelement ermöglicht. Ebenso ist es vorgesehen, dass die Verriegelungseinrichtung durch Drücken entriegelt und durch Loslassen verriegelt. Eine mehrmalige Betätigung ist dann nicht notwendig.

Insbesondere bei einer Handgelenksprothese ist es vorteilhaft, wenn die Verriegelungsvorrichtung parallel zur Schwenkachse verschieblich gelagert ist, so dass eine mehr oder weniger unauffällige Betätigung an dem Handgelenk möglich ist. Diese Betätigung ist im Wesentlichen unabhängig von der Stellung der Kunsthand zu der Basis möglich, da die Kunsthand die Zugänglichkeit in keiner Weise behindert.

Nachfolgend wird ein Ausführungsbeispiel der Erfindung anhand der beigefügten Figuren näher erläutert. Es zeigen:
- Figur 1 -: eine Explosionsdarstellung eines Prothesengelenks;
- Figur 2 -: eine Schnittdarstellung parallel zur Schwenkachse;
- Figur 3 -: eine Schnittdarstellung senkrecht zur Schwenkachse;
- Figur 4 -: eine Einzeldarstellung einer Verriegelungseinrichtung;
- Figur 5 -: eine Explosionsdarstellung einer Variante; sowie
- Figur 6 -: eine Detaildarstellung der Figur 5.

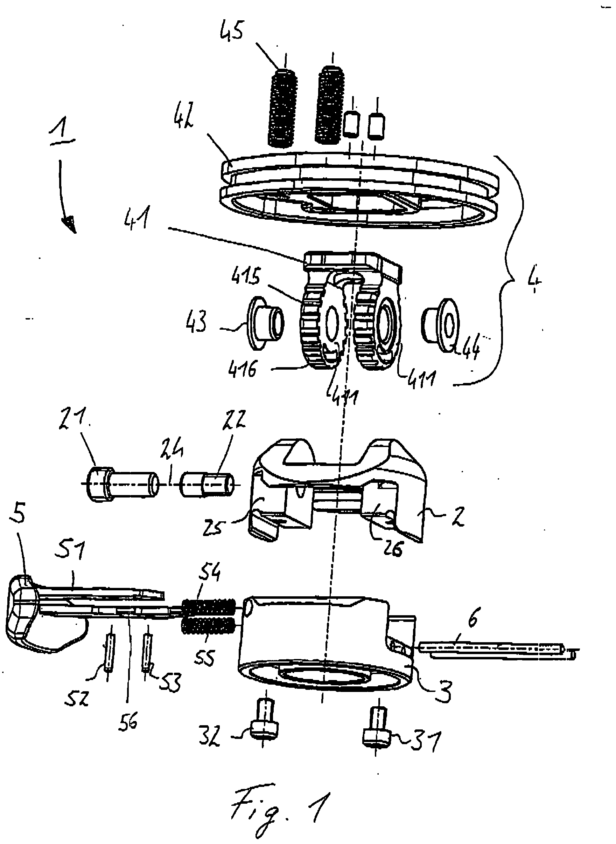

In der Figur 1 ist in Explosionsdarstellung ein Prothesengelenk 1 mit einer Basis 2 und einer gelenkig um eine Schwenkachse 24 befestigten Aufnahmeeinrichtung 4 gezeigt. Die Aufnahmeeinrichtung 4 besteht aus einem Zahnsegmentteil 41, das zwei parallel zueinander ausgerichtete Zahnsegmente 411 aufweist. Diese sind über eine Brücke miteinander verbunden. Auf dieser Brücke ist eine Platte 42 befestigt, auf der ein weiteres Prothesenteil oder eine weitere Protheseneinrichtung montiert werden kann. Beispielsweise kann an den Gewindestiften 45 eine nicht dargestellte Kunsthand angeordnet werden. In den parallelen Zahnsegmenten 411 sind Lagerbohrungen eingebracht, in die Buchsen 43, 44, beispielsweise Kunststoffbuchsen, einführbar sind.

Die Aufnahmeeinrichtung 4 wird in die Basis 2 dergestalt eingeführt, dass die Buchsen 43, 44 mit entsprechenden Bohrungen in Lagerböcken 25, 26 fluchten. Anschließend werden Achsbolzen 21, 22 in die Bohrungen eingeschraubt. Die Achsbolzen 21, 22 bilden die Laufflächen für die Lagerbuchsen 43, 44 aus.

In den Zahnsegmenten 411 sind Ausnehmungen 415 und Einkerbungen 416 vorgesehen, deren Funktion und Wirkungsweise nachfolgend erklärt werden wird.

Die Lagerböcke 25, 26 umschließend ist eine Verriegelungseinrichtung 5 mit Verriegelungszungen 51 vorgesehen, die Vorsprünge 56 aufweisen, die in Richtung auf die Ausnehmungen 415 der Zahnsegmente 411 vorstehen.

In der entriegelten Stellung sind die Vorsprünge 56 in den Verriegelungszungen 51 außer Eingriff mit den Zahnsegmenten 411 und ragen in den Zwischenraum zwischen den beiden Zahnsegmenten 411 zusätzlich hinein, so dass jedes der beiden Zahnsegmente 411 unabhängig von der Verschieberichtung der Verriegelungseinrichtung 5 bzw. der Verriegelungszungen 51 in Eingriff mit den Vorsprüngen 56 treten kann, die in die Ausnehmungen 415 eingreifen.

In die Verriegelungszungen 51 sind Zylinderstifte 52, 53 eingeführt, die sich an Druckfedern 54, 55 abstützen. Die Druckfedern 54, 55 sind in einem Lagerraum zwischen der Basis 2 und der Kopplungseinrichtung 3 gelagert. In der Kopplungseinrichtung 3 sind Bohrungen oder Führungen für ein Rückhalteelement 6, vorliegend zwei elastische Rundschnüre, ausgebildet, die im Wesentlichen parallel zu der Schwenkachse 24 orientiert sind. Das Rückhalteelement 6 kann auch als geschlossenes, elastisches Band ausgebildet sein. Im montierten Zustand greift das Rückhalteelement 6 in die Einkerbungen 416 auf der der Platte 42 abgewandten Seite der Zahnsegmente 411 ein und stützt sich elastisch dagegen ab. Die Basis 2 und das Kopplungselement 3 werden über zwei Schrauben 31, 32 miteinander verbunden.

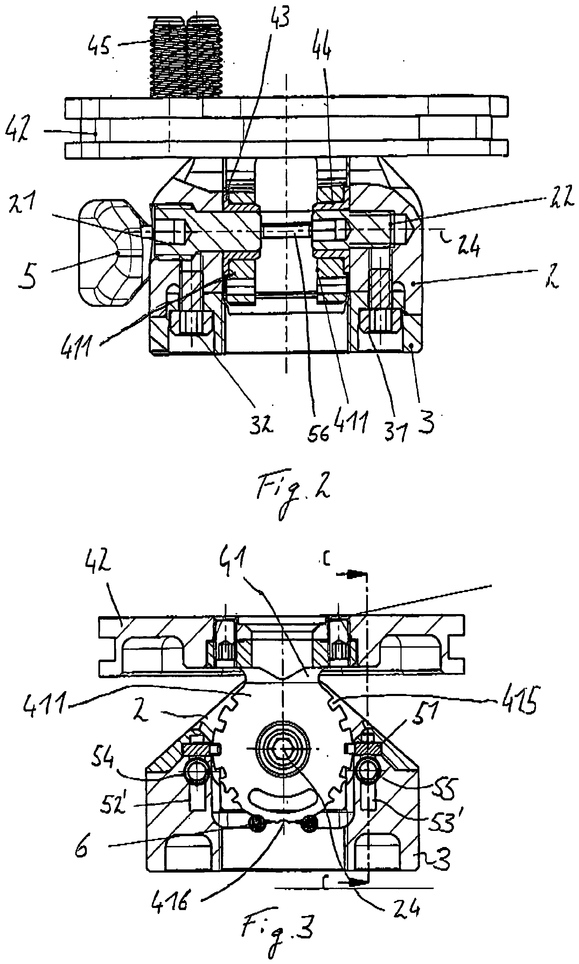

Die Figur 2 zeigt in Schnittansicht das Prothesengelenk 1 im montierten, verriegelten Zustand. Die Basis 2 ist über die Schrauben 31, 32 mit dem Kopplungselement 3 verbunden. Das Zahnsegmentteil 41 ist mit der Platte 42 verschraubt und/oder verklebt. In der Figur 2 ist die Lagerung der Achsbolzen 21, 22 in den Lagerbuchsen 43, 44 innerhalb der Zahnsegmente 411 zu erkennen. Die Achsbolzen 21, 22 sind in die Basis 2 eingeschraubt. Zwischen den Achsbolzen 21, 22 ist ein Freiraum ausgebildet, in dem der jeweils mittlere Vorsprung 56 der Verriegelungszungen 51 hineinragt. In der Figur 2 ist verriegelte Stellung gezeigt, in der der Vorsprung 56 in eine Ausnehmung 415 eingreift. Die Platte 42 ist dann nicht um die Schwenkachse 24 in einem beschränkten Winkel, ausgehend von der dargestellten Ausgangsposition ungefähr ± 60 °, frei verschwenkbar.

In der Figur 3 ist in einem Schnitt senkrecht zur Schwenkachse 24 und der Eingriff der Rückhalteelemente 6 in die Einkerbungen 416 dargestellt. Die Rückhalteelemente 6 können als elastische Stäbe oder als eine Rundschnur ausgebildet sein. Das oder die Rückhalteelemente 6 sind in Richtung auf die Zahnsegmente 41 vorgespannt, in dem vorliegenden Ausführungsbeispiel in Radialrichtung vorgespannt, so dass eine Haltekraft auf die Aufnahmeeinrichtung 4 ausgeübt wird. Diese Haltekraft wird durch Einkerbungen 416, die auf der Unterseite der Zahnsegmente 411 ausgebildet sind, vergrößert, so dass die Rückhalteelemente 6 mit einer relativ geringen Vorspannung montiert werden können. Aufgrund der gleichmäßig zueinander beabstandeten Einkerbungen 416 oder Rasteinkerbungen können verschiedene Raststufen eingestellt werden, bevorzugt in gleichmäßigen Winkelabständen. Die Rasteinkerbungen 416 können den gleichen Abstand wie die Verriegelungsausnehmungen 415 aufweisen und zueinander ausgerichtet angeordnet sein, so dass in jeder Stellung der Aufnahmeeinrichtung 4 im entriegelten Modus eine Verriegelung durch axiales Einschieben der Verriegelungseinrichtung 5 erfolgen kann.

In der Figur 3 sind Freiräume 52', 53' zu erkennen, in denen die vor der Schnittebene liegenden Zylinderstifte 52, 53 bewegbar sind. Die Zylinderstifte 52, 53 sind über die Druckfedern 54, 55 mit einer Rückstellkraft beaufschlagt, so dass die Verriegelungseinrichtung 5 stets in einer Richtung federbelastet ist. In der Ausgangsstellung befindet sich die Verriegelungseinrichtung 5 in der verriegelten Stellung und wird dort gehalten. In der Verriegelungsstellung erfolgt eine formflüssige Festlegung der Aufnahmeeinrichtung 4 durch den Eingriff der Vorsprünge 56 in die Verriegelungsausnehmungen 415. Wird Druck entgegen der Federkraft auf die Verriegelungseinrichtung 5 ausgeübt, werden die Vorsprünge 56 aus den Verriegelungsausnehmungen 415 herausbewegt und ermöglichen eine Verschwenkung der Aufnahmeeinrichtung 4 bzw. der daran befestigten Kunsthand um die Schwenkachse 24. Wird die Verriegelungseinrichtung 5 losgelassen, federt diese aufgrund der Rückstellkraft der Druckfedern 54, 55 zurück und bringt die Vorsprünge 56 wieder in Eingriff mit den Verriegelungsausnehmungen 415. Alternativ kann ein sogenanntes "Push-Push-Element" vorgesehen sein, dass durch zweimaliges Betätigen wieder in die Ausgangsstellung gebracht wird.

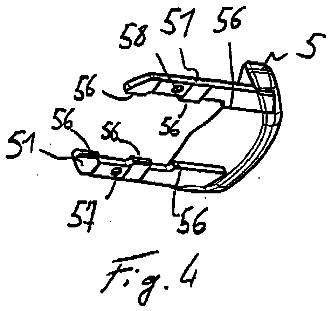

Die Figur 4 zeigt eine Verriegelungseinrichtung 5 mit den Verriegelungszungen 51 und den Vorsprüngen 56 in Einzeldarstellung. In die Bohrungen 57, 58 können die Zylinderstifte 52, 53 eingeführt werden.

Alternativ zu der dargestellten Ausführung mit einer direkten Kopplung eines elastischen Rückhalteelementes 6 mit den Zahnsegmenten 41 bzw. der Aufnahmeeinrichtung 4 kann ein Kontaktteil aus einem harten oder verschleißfesten Werkstoff zwischen einem elastischen Element und den Zahnsegmenten 41 bzw. den Einkerbungen 416 angeordnet werden. Dadurch kann das Übergleiten der Rückhalteelemente 6 bzw. der Kontaktteile einerseits erleichtert und andererseits die Haltbarkeit des Prothesengelenkes erhöht werden.

Ebenfalls ist es möglich, statt einer radial wirkenden Haltekraft eine axial wirkende Haltekraft aufzubringen, beispielsweise, indem in Ausnehmungen in den Seitenflächen der Zahnsegmente 41 Rastausnehmungen oder Vorsprünge eingearbeitet sind, die mit den Rückhalteelementen in Eingriff treten. Beispielsweise kann eine federbelastete Kugel in entsprechend angeordnete, kalottenförmige Ausnehmungen eingreifen.

Durch das erfindungsgemäße Prothesengelenk wird ein unkontrolliertes Herunterfallen einer Protheseneinrichtung nach dem Entriegeln verhindert, ohne dass die Verstellbarkeit wesentlich beeinträchtigt wird. Es kann vorgesehen sein, die Rastelemente außer Eingriff bringbar zu gestalten, so dass für den Fall, dass eine spontane Verstellung ohne das Aufbringen einer zusätzlichen Verstellkraft gewünscht ist, möglich ist. Ebenfalls ist es grundsätzlich möglich, die Vorspannung und damit die Haltekraft einzustellen, um sie auf die persönlichen Wünsche des Prothesenträgers und die sich gegebenenfalls verändernden Gegebenheiten der Protheseneinrichtung anpassen zu können.

Das Prothesengelenk kann entweder unmittelbar an einem Befestigungsmittel zur Festlegung an einem Körperglied oder an einem weiteren Prothesenteil angebracht sein, beispielsweise an einer Unterarmprothese.

Neben dem Einsatz als Prothesenhandgelenk können auch andere Gelenkeinrichtungen vorgesehen sein, beispielsweise als Knie- oder Ellenbogengelenk. Ebenfalls ist eine Einsatz bei einer motorisch angetriebenen Prothese möglich, um den Energieverbrauch zu reduzieren. Die Haltekraft im entriegelten Zustand kann dann so gering gewählt werden, dass der Antrieb nur eine geringfügig höhere Leistung bereitstellen muss- Dieser Mehraufwand wird durch den Verzicht auf motorisch gehaltenen Protheseneinrichtungen kompensiert. Die Steuerung des Antriebes kann über myoelektrische Signale erfolgen.

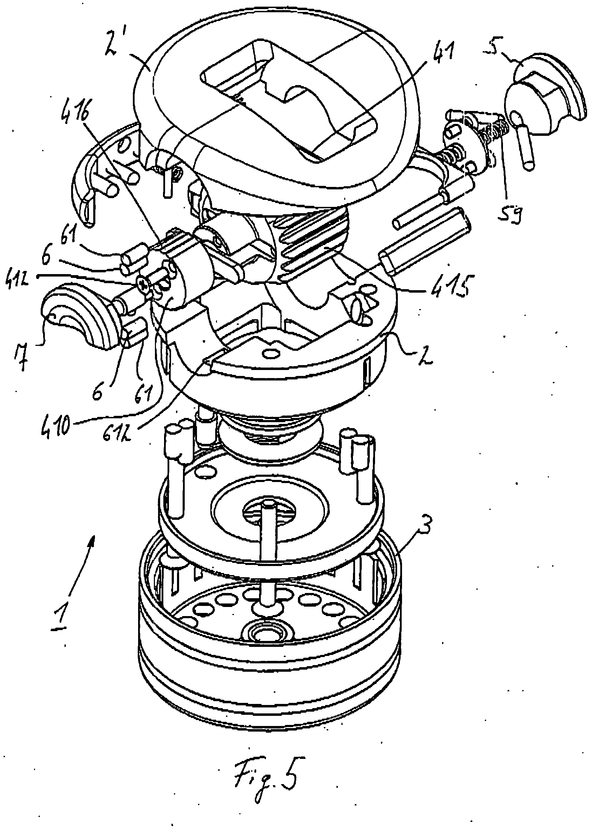

In der Figur 5 ist eine Variante eines Prothesengelenkes 1 in Explosionsdarstellung gezeigt, die Rückhalteelemente 6 sind dabei als Stahlstifte ausgebildet, die über eine elastische Schnur 61 bzw. ein elastisches Element elastisch gegen eine Hülse 410 vorgespannt ist und diese mit einer Haltekraft beaufschlagt. Die Hülse 410 ist über Schrauben 412 mit dem Zahnsegmentteil 41 verbunden und überträgt somit die radial aufgebrachte Haltekraft von der Hülse 410 auf die Aufnahmeeinrichtung 4. Die zweitteilige Ausgestaltung des Rückhalteelementes in Gestalt eines Stahlstiftes 6 und eines elastischen Elementes 61 erfolgt zur Erhöhung der Haltbarkeit und Verschleißminimierung.

Die elastische Schnur 61 bzw. das elastische Element ist in Ausnehmungen 612, von denen nur eine dargestellt ist, in der Basis 2 gelagert, entsprechend ist eine weitere Ausnehmung in dem Oberteil 2' der Basis 2 angeordnet.

Die Hülse 410 ist ebenso wie die Rückhalteelemente 6 aus Stahl gefertigt, um eine verschleißminimierte Werkstoffpaarung bereitzustellen. Alternativ können die Einkerbungen 416 auch unmittelbar an dem Zahnsegmentteil 41 ausgebildet sein.

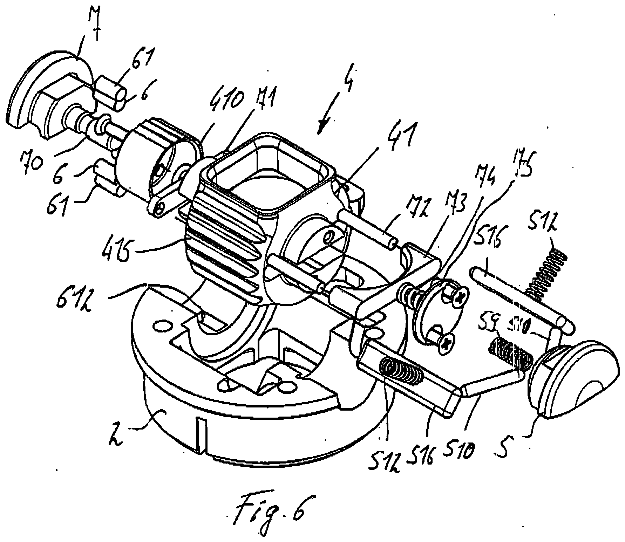

In der Figur 6 ist das Prothesengelenk 1 gemäß Figur 5 in einer Detaildarstellung ohne Oberteil 2' der Basis 2 dargestellt. Darin ist zu erkennen, dass das Zahnsegmentteil 41 einteilig ausgebildet ist und darüber hinaus die Aufnahmeeinrichtung 4 für ein Kopplungselement mit der nicht dargestellten Kunsthand ausbildet. Ein Ver- und Entriegelungsmechanismus ermöglicht ein schnelles, formflüssiges Ver- und Entriegeln eines Adapters einer Kunsthand in der als Zahnsegmentteil 41 ausgebildeten Aufnahmeeinrichtung 4. Die Verriegelung der Aufnahmeeinrichtung 4 entgegen einer Verschwenkbewegung erfolgt über die als Stege 516 ausgebildeten Verriegelungseinrichtungen, die entgegen einer durch Fedem 512 aufgebrachten Rückstellkraft über Stifte 510 aus der voreingestellten Eingriffsstellung herausgeschwenkt werden können. Die Stege 516 sind in der Basis 2 federnd gelagert. Die Stifte 510 liegen an einer kegelig ausgebildeten Betätigungsfläche der Verriegelungseinrichtung 5 an. Die Verriegelungseinrichtung 5, vorliegend als Drucktaster ausgebildet, ist über eine Druckfeder 59 in einer Ausgangsstellung gehalten, in der die Stege 516 in Eingriff mit den Verriegelungsausnehmungen 415 stehen. Wird der Taster 5 gedrückt, werden die Stifte 510 entgegen der Federkraft gegen die Stege 516 gedrückt und verschwenken diese außer Eingriff, wird der Taster 5 losgelassen, erfolgt eine umgekehrte Bewegung.

Zur möglichst schnellen Ver- oder Entriegelung der Kunsthand mit der Aufnahmeeinrichtung 4 ist ein weiterer Drucktaster 7 vorgesehen, der über eine Achse 70, die durch die Hülse 410 hindurchgeht, mit einem Kraftübertragungselement 71 gekoppelt ist. Dieses Kraftübertragungselement 71 ist über Stifte 72 mit einem Formschlusselement 73 gekoppelt, das verschiebbar und entgegen einer Federkraft in der Aufnahmeeinrichtung 4 gelagert ist. Wird der Taster 7 betätigt, wird das Formschlusselement 73 entgegen der Kraft der Feder 74 in Richtung auf das verschraubte Widerlager 75 verschoben und gibt die hier nicht dargestellte Kunsthand frei. Zum Verriegeln einer Kunsthand wird der Taster 7 gedrückt gehalten, wodurch die Aufnahmeeinrichtung 4 zum Einschieben eines Adapters einer Kunsthand aufbereitet wird. Nach dem einschieben und Lösen des Tasters 7 wird der Adapter aufgrund der Rückstellkraft der Druckfeder 74 automatisch verriegelt. Ebenfalls ist es vorgesehen, dass der Adapter in die Aufnahmeeinrichtung 4 eingesteckt wird, dass dieser selbsttätig das Formschlusselement 73 verschiebt und anschließend formschlüssig in der Aufnahmeeinrichtung 5 verriegelt gehalten wird.

## Patentansprüche

1. Prothesengelenk, insbesondere für eine Handprothese, mit einer Basis (2) und einem der Basis zugeordneten Kopplungselement (3) zur Befestigung an einem Körperglied oder einem Prothesenteil sowie mit einer mit der Basis um zumindest eine Achse (24) verschwenkbar verbundenen Aufnahmeeinrichtung (4) für eine Protheseneinrichtung, insbesondere eine Kunsthand, wobei eine Verriegelungseinrichtung (5, 51) die Aufnahmeeinrichtung (4) gegenüber der Basis (2) blockiert, wobei ein Rückhalteelement (6) elastisch gegen die Aufnahmeeinrichtung (4) vorgespannt ist und diese nach einer Entriegelung der Verriegelungseinrichtung (5, 51) mit einer Haltekraft beaufschlagt, **dadurch gekennzeichnet, dass** die Aufnahmeeinrichtung (4) Rastelemente (416) aufweist, in die das Rückhalteelement (6) eingreift.

2. Prothesengelenk nach Anspruch 1, **dadurch gekennzeichnet, dass** die Vorspannung des Rückhalteelementes (6) dergestalt ist, dass die Aufnahmeeinrichtung (4) entgegen der Schwerkraft in der entriegelten Stellung gehalten wird.

3. Prothesengelenk nach Anspruch 1, **dadurch gekennzeichnet, dass** die Rastelemente (416) als Ausnehmungen, Einkerbungen oder Vorsprünge ausgebildet sind.

4. Prothesengelenk nach einem der voranstehenden Ansprüche, **dadurch gekennzeichnet, dass** das Rückhaltelement (6) als elastisches Band, insbesondere als Rundschnur ausgebildet ist.

5. Prothesengelenk nach einem der Ansprüche 1 bis 3, **dadurch gekennzeichnet, dass** das Rückhalteelement (6) ein formstabiles Kontaktteil ist, das an der Aufnahmeeinrichtung (4) anliegt und in Richtung auf die Aufnahmeeinrichtung (4) elastisch gelagert ist.

6. Prothesengelenk nach Anspruch 5, **dadurch gekennzeichnet, dass** das Kontaktteil (6) als Lagerstift, insbesondere als Stahlstift ausgebildet ist.

7. Prothesengelenk nach einem der voranstehenden Ansprüche, **dadurch gekennzeichnet, dass** die Verriegelungseinrichtung (5, 51) als Push-Push-Element ausgebildet ist, das die Aufnahmeeinrichtung (4) formschlüssig verriegelt.

8. Prothesengelenk nach Anspruch 7, **dadurch gekennzeichnet, dass** die Verriegelungsvorrichtung (5, 51) parallel zur der Schwenkachse (24) verschiebbar gelagert ist.

## Claims

1. Prosthesis joint, particularly for a hand prosthesis, with
a base (2) and
a coupling element (3) associated to the base for attaching the base to a body part or a prosthesis part and
a receiving device (4) for a prosthesis device, in particular an artificial hand, the receiving device being pivotally attached to the base around at least one axis (24), wherein
a locking device (5, 51) blocks the receiving device (4) against the base (2), wherein
a retention element (6) is elastically prestressed towards the receiving device (4) and applies a holding force to the receiving device after unlocking the locking device (5, 51),
**characterised in that** the receiving device (4) comprises notch elements (416) that engage with the retention element (6).

2. Prosthesis joint according to claim 1, **characterised in that** the prestressing of the retention element (6) is constituted to hold the receiving device (4) against gravitational force in the unlocked position.

3. Prosthesis joint according to claim 1, **characterised in that** the notch elements (416) are formed as recesses, indentations or protrusions.

4. Prosthesis joint according to one of the preceding claims, **characterised in that** the retention element (6) is formed as an elastic band, particularly a round cord.

5. Prosthesis joint according to one of the claims 1 to 3, **characterised in that** the retention element (6) is an inherently stable contact member that abuts against the receiving device (4) and is elastically mounted towards the receiving device (4).

6. Prosthesis joint according to claim 5, **characterised in that** the contact member (6) is formed as a hinge pin, particularly a steel pin.

7. Prosthesis joint according to one of the preceding claims, **characterised in that** the locking device (5, 51) is formed as a push-push-element that positively locks the receiving device (4).

8. Prosthesis joint according to claim 7, **characterised in that** the locking device (5, 51) is movably mounted parallel to the pivot axis (24).

## Revendications

1. Articulation prothétique, en particulier pour une prothèse de la main, comprenant une base (2) et un élément de couplage (3) associées à la base, destiné à être fixé sur un membre corporel ou sur une partie de prothèse, ainsi qu'un dispositif récepteur (4) reliée à la base avec possibilité de pivotement autour d'au moins un axe (24), pour un système de prothèse, en particulier une main artificielle, dans laquelle un dispositif de verrouillage (5, 51) bloque le dispositif récepteur (4) par rapport à la base (2), et dans laquelle un élément de retenue (6) est précontraint élastiquement contre le dispositif récepteur (4) et sollicite celui-ci, après un déverrouillage du dispositif de verrouillage (5, 51), avec une force de maintien,
**caractérisée en ce que** le dispositif récepteur (4) comprend des éléments à enclenchement (416) dans lesquels s'engage l'élément de retenue (6).

2. Articulation prothétique selon la revendication 1, **caractérisée en ce que** la précontrainte de l'élément de retenue (6) est telle que le dispositif récepteur (4) est maintenu dans la position déverrouillée à l'encontre de la gravité.

3. Articulation prothétique selon la revendication 1, **caractérisée en ce que** les éléments à enclenchement (416) sont réalisés sous forme d'évidements, d'encoches ou de saillies.

4. Articulation prothétique selon l'une des revendications précédentes, **caractérisée en ce que** l'élément de retenue (6) est réalisé sous forme de bande élastique, en particulier sous forme de cordon rond.

5. Articulation prothétique selon l'une des revendications 1 à 3, **caractérisée en ce que** l'élément de retenue (6) est une pièce de contact à forme stable, qui s'applique contre le dispositif récepteur (4) et qui est montée élastiquement en direction du dispositif récepteur (4).

6. Articulation prothétique selon la revendication 5, **caractérisée en ce que** la pièce de contact (6) est réalisée sous forme de tige de montage, en particulier de tige en acier.

7. Articulation prothétique selon l'une des revendications précédentes, **caractérisée en ce que** le dispositif de verrouillage (5, 51) est réalisé comme un élément poussé-poussé ("push-push"), qui verrouille le dispositif récepteur (4) par coopération de formes.

8. Articulation prothétique selon la revendication 1, **caractérisée en ce que** le dispositif de verrouillage (5, 51) est monté avec possibilité de déplacement parallèlement à l'axe de pivotement (24).
